# EUROPEAN PATENT SPECIFICATION

(11) **EP 2 934 420 B1**
(45) Date of publication and mention of the grant of the patent: **07.02.2018**
(21) Application number: 12816052.0
(22) Date of filing: 21.12.2012
(51) Int. Cl.: A61J 1/06, A61M 5/24, B65D 51/00

(54) **PRIMARY PACKAGING FOR STORAGE AND/OR ADMINISTRATION OF MEDICAL OR PHARMACEUTICAL COMPOUNDS**
PRIMÄRVERPACKUNG ZUR LAGERUNG UND/ODER VERABREICHUNG MEDIZINISCHER ODER PHARMAZEUTISCHER VERBINDUNGEN
EMBALLAGE PRIMAIRE POUR LE STOCKAGE ET/OU L'ADMINISTRATION DE COMPOSÉS PHARMACEUTIQUES OU MÉDICAUX

(43) Date of publication of application: 28.10.2015
(73) Proprietor: Skufca, Peter, 78315 Radolfszell (DE)
(72) Inventor: Skufca, Peter, 78315 Radolfszell (DE)
(74) Representative: Winter, Brandl, Fürniss, Hübner, Röss, Kaiser, Polte - Partnerschaft mbB
(86) International application number: PCT/EP2012/076601
(87) International publication number: WO 2014/026721

(56) References cited:
- EP-A1- 2 535 073
- EP-A2- 0 312 035
- DE-B- 1 207 043
- JP-U- S58 126 840
- US-A- 3 413 975

## Description

The present invention relates to a primary packaging for storage and/or administration of medical or pharmaceutical compounds.

From the state of the art, primary packaging for storage and/or administration of medical or pharmaceutical compounds are known which are intended to contain and protect those compounds and which are or may be in direct contact with them. According to the WHO (World Health Organization) "Guidelines on packaging for pharmaceutical Products", issued in the WHO Technical Report Series, No. 902, 2002, such a primary packaging must protect the pharmaceutical or medical products against all adverse external influences that may affect its quality or potency such as, for example, light, moisture, oxygen, biological contamination or mechanical damage. In particular, such a primary packaging must not interact physically or chemically with the contended medical or pharmaceutical compounds in any way that would alter their quality.

A primary packaging for the intended use usually comprises a container which holds or is intended to contain and protect medical or pharmaceutical compounds, and a closure which covers the compounds or which is intended to seal the container. Those containers and closures may be intended for single-use or multi-use. According to the above-mentioned WHO Guidelines, a primary packaging must protect the contents from extraneous matter, from loss of the substance, and from efflorescence, deliquescence or evaporation under normal conditions of handling, shipment or storage.

For the use as a primary packaging, several containers are well known from the state of the art, for example, from US 3,413,975, EP 2 535 073 A1, DE 1 207 043 A or EP 0 312 035 A2. Also JPS58126840U discloses a primary packaging known from state of the art. Containers which are often used for the purpose of packaging medical or pharmaceutical compounds are, for example, vials, ampoules, cartridges, bottles or (pre-filled) syringes. Those containers may be manufactured in glass or in plastic. Of particular importance in such usage are ampoules which are single-use containers sealed by fusion and to be opened exclusively by breaking, vials which are single-use or multi-use containers closed by a stopper and/or a overseal, and syringes which are cylindrical devices with a cannula-like nozzle and with or without a fixed needle and a movable piston. Further, for the use as a primary packaging several closures are well known from the state of the art. For example, such a closure may be made from elastomeric materials and from polyethylene or polypropylene. In this context it is notable that such closures usually may have different shapes and sizes, e.g. stoppers for infusion or injection bottles or plungers for prefilled syringes, depending on the type of container.

Those containers and closures are specified, inter alia, by the standards on packaging issued by the International Organization for Standardization (ISO). In particular, relevant ISO standards are ISO 8362-1, 8362-2, ISO 11040-4 and ISO 11040-5.

The choice or assembling, respectively, of the primary packaging depends, among other things, on the degree of protection required as well as the compatibility with the contents on the one hand, and the filling method, the production costs, the presentation or the form of administration format as well as the convenience of the packaging for the user, e.g. the size, weight, method of opening, on the other hand. Regarding the filling method, it is notable that filling facility is usually laid out for only one type of packaging. That is, for example, a vial can only be filled in a vial-filling facility and a syringe can only be filled in a syringe-filling facility.

Further, it is known that the individual components of such a packaging, including a container and a closure, but also the combination thereof, have to pass through a variety of tests to be approved or certificated as a primary packaging. Such tests are complex and costly. Therefore, a primary packaging for a specific medical or pharmaceutical compound is often set or fixed in the early stage development of the compound and is changed in the further course of development only if necessary. Accordingly, a disadvantage of a conventional primary packaging according to the state of the art is its inflexibility with regard to a change of its structure, specific form of administration, etc. and/or of its individual components during development.

It is also known that, for example vaccines filled in e.g. pre-filled syringes, are produced only seasonally as their typical applications, such as vaccination, is carried out only in certain seasons. Nevertheless, the filling facilities and filling lines for the syringes have to be kept throughout the year, since they cannot be dismantled easily and built up again. Consequently, the maintenance costs of the filling facilities used for filling syringes are high or they are shut down for a period of time during the year. This has a negative impact on the production costs of medical or pharmaceutical products.

It would therefore be desirable to provide a more flexible primary packaging that overcomes such drawbacks. For this purpose, several solutions have been proposed in the prior art.

For example, from EP 0 298 585 A1 a pre-filled syringe is known, which is based upon the use of a vial containing a medical compound in order to be able to fill the vial by using a conventional filling facility and technique for vials. The vial has an open bottom which is closed by a piston that can be coupled with a plunger. By an adapter cap having an internal needle and an external connection for a needle that is placed over a closure of the vial, the system may be converted into a pre-filled syringe. A disadvantage of this system is that the vial can only be filled by using filling facilities used for filling vials. Moreover, due to an open bottom of the vial, this vial can only be used in connection with the proposed system.

In US 4 568 336 A, another pre-filled syringe is disclosed, which has a tubular barrel that can be filled at either end. Both ends of the syringe barrel may be sealed with standard vial closures and crimped aluminum seals. One closure means for the barrel is a piston and the other closure means is adapted to receive a needle. A disadvantage of the proposed syringe is that it can only be used as a pre-filled syringe. Document JPS58126840U discloses a primary packaging in accordance with the preamble of claim 1.

The object of the present invention is to improve a primary packaging which enables a user to easily handle it as a syringe and which allows the container to be conveyed through a conventional filling facility for standardized syringes.

This object is solved by a primary packaging with the features of claim 1.

Advantageous embodiments of the invention are specified in dependent claims.

A primary packaging for storage and/or administration of medical or pharmaceutical compounds according to the present invention comprises a container having a predetermined filling volume for receiving a medical or pharmaceutical compound. The container is permanently closed at a first end and has an integrally formed, radially extending circumferential flange portion at an open second end. The container has a cylindrical barrel that extends between the first end and the flange portion. The primary packaging according to the present invention further comprises a closure element which is preferably adapted to tightly fit on the flange portion and/or to tightly fit into the barrel in order to seal the open second end of the container (after filling of the container).

As described above, for the purpose of the disclosure, "primary packaging" is taken to mean any packaging which is in direct physical contact with the medical or pharmaceutical compound to be filled. Further, the term "container" is used as a generic term for a container which is made of materials, such as glass and plastic, suitable for medical or pharmaceutical compounds. Preferably, a "cylindrical barrel" is to be understood as a hollow cylindrical barrel which is formed, in terms of its shape and dimensions, as far as appropriate along the lines of the specifications of the ISO 11040-4 standard. A "flange portion" according to the present invention is preferably taken to mean a flange which is formed, in terms of its cross-sectional shape, as far as appropriate along the lines of the specifications of the ISO 8362-1 standard, and on which another object, i.e. a closure element, can be safely attached if necessary. A "standardized syringe" is to be understood as a syringe which complies with the specifications of the aforementioned ISO 11040-4 standard. Similarly, a "standardized vial" is taken to mean a vial that complies with the specifications of the aforementioned ISO 8362-1 standard.

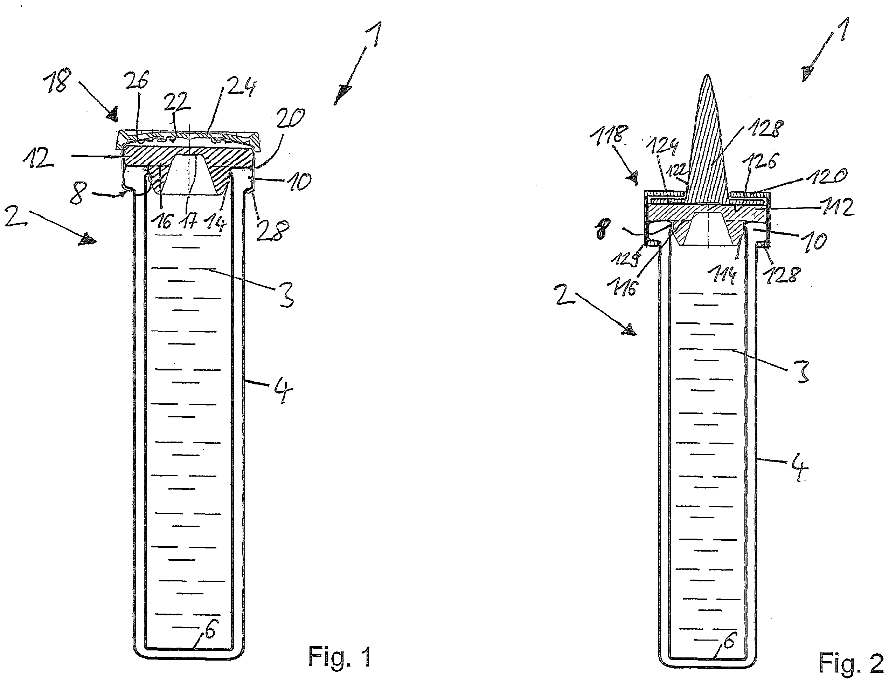

Preferably the cylindrical barrel complies, in terms of its inner diameter, outer diameter and wall thickness, with the relevant specifications of the above-mentioned ISO 11040-4 standard for a suitable specific standardized nominal volume. Said specific standardized nominal volume may correspond to or may be close to, i.e. may slightly differ from, the predetermined filling volume of the container, i.e. is a suitable one of the various nominal volumes considered in the ISO 11040-4 standard. In particular, the cylindrical barrel may be formed to meet the barrel diameters d1 and d2 and the barrel wall thickness s1 as indicated in Figure 1 and Table 1 of the ISO 11040-4 standard for a specific standardized nominal volume of a standardized syringe. Depending on the predetermined filling volume, the length of the barrel may conform to the length I1 or total length l of a standardized syringe as indicated in Figure 1 and Table B.1 of ISO 11040-4 standard, or may vary within a range defined by the length l1 and the total length l as aforementioned, or may even be different from the specifications of the ISO 11040-4 standard. Generally, the length of the cylindrical barrel is defined and set so that the predetermined filling can be achieved with the inner diameter, outer diameter, and wall thickness adopted from the ISO 11040-4 standard for the specific nominal volume as above explained. If the predetermined filling volume matches a standardized nominal volume of a standardized syringe, the standardized nominal volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this standardized or specific nominal volume. In this case, the length of the barrel may meet the length l1 indicated as indicated in Figure 1 and Table 1 of the ISO 11040-4 standard for the standardized nominal volume. If the predetermined filling volume differs from any of the nominal volumes set by the ISO 11040-4 standard, any suitable nominal volume close to the predetermined filling volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this suitable or specific nominal volume. Even if the predetermined filling volume matches a standardized nominal volume of a standardized syringe, however, any other suitable standardized nominal volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this other standardized or specific nominal volume. For example, if the predetermined filling volume is 1 ml the cylindrical barrel may meet the outer diameter d1, inner diameter d2, and wall thickness s1 of a 1 ml syringe in a long version, with d1 being 8.15 mm ±0.1 mm, d2 being 6.35 mm ±0.1 mm, and s1 being approximately 0.9 mm (cf. ISO 11040-4, Tables 1 and B.1 for a nominal volume of 1 ml). In this case, the barrel length may meet the length l1 of the 1 ml syringe in the long version, being 54 mm ±0.5 mm, with the specific nominal volume being 1 ml, With the same predetermined filling volume of 1 ml, however, the cylindrical barrel may alternatively meet the outer diameter d1 (= 10.85 mm ±0.1 mm), inner diameter d2 (= 8.65 mm ±0.2 mm), and wall thickness s1 (≈ 1.1 mm) of a 1 ml syringe in a short/standard version (cf. ISO 11040-4, Tables 1 and B.1 for a nominal volume of 1 ml) with the specific nominal volume being 1 ml, or may meet the outer diameter d1 (= 6.85 mm ±0.1 mm), inner diameter d2 (= 4.65 mm ±0.1 mm), and wall thickness s1 (≈ 1.1 mm) of a 0.5 ml syringe (cf. ISO 11040-4, Tables 1 and B.1 for a nominal volume of 0.5 ml) with the specific nominal volume being 0.5 ml. In these alternative cases, the barrel length is appropriately adjusted so as to ensure that the container provides the predetermined filling volume.

Further, the flange portion put on top of the cylindrical barrel preferably forms, in a circumferential direction, a continuous circular flange in line with the flange of a conventional vial according to the ISO 8362-1 standard. Insofar, the flange is also in line with a form B of a finger flange of a standardized syringe (cf. ISO 11040-4, Figure 1, Form B). Additionally, the flange portion may be formed to comply, in terms of its cross-sectional shape, with relevant specifications of the above mentioned ISO 8362-1 standard. In particular, the flange portion may meet, in terms of its axial length/height, in terms of its upper inner edge, and/or in terms of its upper end surface, the relevant specifications as indicated in Figure 1, Figure 2, Figure 3 and Table 1 of the ISO 8362-1 standard. Specifically, the axial length/height of the flange may amount to 3.6 mm ±0.2 mm, the bevel angle of the upper inner edge may be approximately 45°, and/or the taper angle of the upper end surface of the flange may be 3° ±2° (cf. ISO 8362-1, Figures 1 to 3). Adopting the aforementioned length dimensions and angles may be useful in order to enable the flange portion of the container to cooperate with a conventional closure element as specified in the ISO 8362-2 standard. While the above mentioned 3.6 mm ±0.2 mm adopted from the ISO 8362-1 standard are preferable, the axial length/height of the flange portion may slightly differ from this standardized dimension as long as the flange portion still meets the following two functions: first, the flange portion shall enable handling and processing the container in conventional filling facilities and with conventional technology used for filling and processing of standardized syringes, i.e. the flange portion shall meet the function of a finger flange of a standardized syringe (cf. ISO 11040-4, Figure 1); and secondly the design of the flange portion shall allow a tight closure of the container by using an appropriate closure element, e.g. a closure element according to the ISO 8362-2 standard of a conventional standardized vial. Further, the flange portion may differ, in terms of its inner diameter, outer diameter and its lower end surface, from the relevant specifications of the ISO 8362-1 standard (cf. ISO 8362-1, Figure 1: diameter d4, diameter d2) in order to enable the flange portion to smoothly match the respective barrel dimensions. In particular, the flange portion may have a radially extending flat lower end surface unlike a standardized vial which has a tapered lower surface (cf. ISO 8362-1, Figures 1 to 3 showing a taper angle of 10° ±5°). A flat lower end surface may facilitate the handling of the container in conventional filling facilities and with conventional technology used for filling and processing of standardized syringes. Generally, however, the lower end surface of the flange portion may be formed with a taper angle as it is known from ISO 8362-1 (cf. Figures 1 to 3 showing a taper angle of 10° ±5°).

Accordingly, unlike a conventional vial the container of a primary packaging according to the present invention has, at its outer surface, no such neck constriction as it seen and specified in the ISO 8362-1 standard (cf. ISO 8362-1, Figures 1 to 3: diameter d3, height h3). On the other hand, the inner surface of the container may be finished at the upper end, i.e. the upper end section oppositely to the flange portion, in line with the finish of any appropriate one of vial models A, B, or C of the ISO 8362-1 standard (cf. ISO 8362-1, Figures 1 to 3).

Further, an inner surface of the container, which is in contact with the medical or pharmaceutical compound stored in the container, may be refined by way of subjecting the inner surface to an appropriate surface finishing process and/or coating process. For example, the inner surface may be refined by diverse plasma treatments or siliconized by baked-on or spray siliconization using silicone oil suspensions or silicone oil. Such treatment is known with conventional prefilled syringes in order to enhance the lubrication property of the inner surface of a syringe barrel. It has to be noted, however, that according to the present invention a surface finishing of the inner surface of the container is not restricted to the use of the primary packaging as a syringe, but is open to any type of use of the primary packaging whether it is as a syringe, vial or ampoule, which types of use will be described in the following.

By the configuration according to the present invention, a modular system for assembling a primary packaging is provided, which is based on a single type of container that preferably meets, in terms of shape and dimensions of the barrel, the relevant specifications of a standardized syringe, and meets, in terms of the cross-sectional shape of the flange portion, the relevant specifications of a standardized vial.

The closure element can be any closure element selected from among a group of variants of closure elements, depending on the compound to be stored and/or on the purpose of administration of the compound, and is made of a material suitable for the usage with medical or pharmaceutical compounds. Preferably the material of the closure element is an elastomeric material, preferably rubber.

By the configuration of a primary packaging according to the present invention, that is the combination of a container which has a body formed as a cylindrical barrel which complies, as far as appropriate, with the relevant specifications of the above mentioned ISO 11040-4 standard of a conventional or standardized syringe and which has a flange portion that complies, in terms of the cross-sectional shape, as far as appropriate with relevant specifications of the above mentioned ISO 8362-1 standard of a conventional vial, the container according to the present invention is advantageously adapted to be filled by standardized or conventional filling facilities and technology used for filling and processing of standardized syringes. Thus, the existing machine capacity of those conventional filling facilities and technology can be used in an advantageous manner. It is noteworthy that the container is permanently closed on one end, unlike the barrel of a conventional standardized syringe, so that the container can also be used as a vial. Further, as above mentioned, unlike a conventional standardized vial the container has no neck constriction between the flange portion and the barrel. According to the present invention, the preferably continuously circular flange portion is directly joined to the barrel.

Another advantage of the present invention originates from the fact that, by meeting the cross-sectional shape of the flange of a standardized vial according to the ISO 8362-1 standard as far as appropriate, the flange portion of the container is manufactured in accordance with the standardized accuracy requirements of a standardized vial according to the ISO 8362-1 standard. Because of this fact, it is possible to use the disclosed container with any closure element to be used with a conventional vial. Further, due to the configuration of the flange portion of the container according to the present invention, the container fulfills all requirements with respect to impermeability of the ISO 8362-1 standard.

In other words, the combination of a container and a closure element in accordance with the present invention can be used as an alternative for a standardized vial in order to store medical or pharmaceutical compounds and, additionally, can be filled and processed by using conventional filling facilities and technology used for standardized syringes. In this way, the primary packaging according to the invention has an advantageous double/multiple function.

According to the present invention, the flange portion is formed to function as a finger flange. This means that the flange portion of the container is formed to function as a finger rest. Due to this configuration it is possible to easily convey the container through a conventional filling facility for standardized syringes. Additionally, the finger flange enables a user to easily grasp the container. Of course, the design of the finger-flange may be agreed between customer and manufacturer in compliance with the above-mentioned ISO standards.

According to the present invention, the closure element is a cylindrical plunger stopper comprising a fitting portion adapted to tightly fit into the barrel of the container.

According to the present invention the primary packaging further comprises a needle extraction system for removal of the medical or pharmaceutical compound, wherein the needle extraction system is adapted to cooperate with the plunger stopper. It is remarkable that in this case the needle extraction system, when cooperating with the plunger stopper, extends outwards from the open second end of the container. For a cooperation of the needle extraction with the closure element formed as a plunger stopper, the needle extraction system and the plunger stopper is provided with appropriate engaging or attachment means which are adapted to fit to each other. By this configuration, the primary packaging according to the invention can be used either as a vial, as an ampoule or as a syringe, while being still producible by a conventional filling facility used for standardized syringes. Here, the filling volume of the barrel may be even larger than in the case of a standard syringe, since no needle extends from the permanently closed first end of the container. Consequently, in this case the barrel can have a length that is larger compared to a standard syringe, while being still producible by a conventional filling facility.

The needle extraction system comprises a plunger rod element that is releasably attachable to the plunger stopper by a snap fit connection or, alternatively, by a screw fit connection. In this way, the primary packaging can be easily converted to a syringe. Contrary to a conventional prefilled syringe, however, the primary packaging when used as a syringe is both filled and extracted via the open second end of the container, i.e. at the same end side of the container. The plunger rod element has a finger flange in order to facilitate the removal of the medical or pharmaceutical compounds stored in the container.

Further, a cannula is fixed to the plunger rod element.

According to a preferred embodiment, a seal can be provided, which at least partially encloses the closure element in a sealing manner, and which can also engage the flange portion of the container. Such a seal can be used as an overseal to secure the closure element, especially if the closure element is made of rubber, to the container in order to maintain the integrity of the sealing under normal conditions of transport, handling and storage during an intended shelf-life of the product.

As was already described hereinbefore, the primary packaging can be assembled by using a modular system in which the individual components, i.e., the container, the various closure elements, the various seals, etc., are approved for usage with pharmaceuticals and in which the individual components are easily exchangeable. Here, the container of the primary packaging can be used either as a vial, as an ampoule or as a syringe, while still being producible by a conventional filling facility for standardized syringes. It is notable that the primary packaging according to the invention comprises a single type of container that is suitable for various closure elements and a needle extraction system. Regarding the needle extraction system, it is also noteworthy that the end side of removal of the medical or pharmaceutical compound from the container is the very same end side as the end side of filling the container.

In the following, advantageous embodiments of the invention will be described in more detail with reference to the below drawings.
- Figure 1: is a schematized sectional side view of an example of a container with a closure element and a seal,
- Figure 2: is a schematized sectional side view of another example of a container with a closure element and a seal,
- Figure 3: is an enlarged, schematized sectional side view of a seal according to Figure 2,
- Figure 4: is a schematized sectional side view of a container with a closure element according to an embodiment of the invention,
- Figure 5: is a schematized sectional side view of a needle extraction system according to the invention,
- Figure 6: is a schematized sectional side view of a container with a closure element and the needle extraction system of Fig. 5, attached to the closure element,
- Figure 7: is a schematized sectional side view of a needle extraction system according to another embodiment of the invention, and
- Figure 8: is a schematized sectional side view of a container with a closure element and the needle extraction system according to Figure 7, attached to the closure element.

Identical components are denoted by identical reference signs throughout the figures.

Figure 1 shows a sectional side view of an example of a primary packaging 1. The primary packaging 1 serves to receive any medical or pharmaceutical compounds for storage and/or administration of the same. The primary packaging 1 is normally in direct contact with these compounds.

The primary packaging 1 comprises a container 2 made of glass or any appropriate plastics material, which has a defined filling volume for receiving the medical or pharmaceutical compounds 3. The container 2 has a cylindrical barrel 4 and is permanently closed at a first end 6 that forms the bottom of the container 2. Further, the container 2 has an open second end 8 that forms the top of the container 2. At the open second end 8, an integrally formed, circumferential flange portion 10 is provided that extends radially outwardly from the barrel 4. This means that the barrel 4 extends between the closed first end 6 and the flange portion 10 in a cylindrical shape.

In the example, the container 2 has an exemplary predetermined filling volume of 1.9 ml. For this purpose, the barrel 4 of the container 2 has an outer diameter of 8.15 mm and an inner diameter of 6.35 mm, as specified by the ISO 11040-4 standard (cf. of ISO 11040-4, Figure 1 and Tables 1 and B.1 for dimensions d1, d2) for a so called long version of a standardized syringe having a nominal volume of only 1 ml. Due to the fact that the first end 4 of the container 2 is permanently closed instead of having a head-shaped end known from a conventional standardized syringe (cf. ISO 11040-4, Figures A.1 to A.3 showing various head designs), for holding a needle cannula, however, the total length of the filling volume of the barrel 4, in the example, may be approximately 64 mm as compared to the total length of the filling volume of the barrel of the aforementioned long version of the conventional standardized syringe, amounting to only 54 mm (cf. ISO 11040-4, Table 1, l1 for a nominal volume of 1 ml) in view of the fact that a conventional standardized syringe needs an unspecified additional amount of length corresponding to the length of the head design of a conventional standardized syringe (cf. ISO 11040-4, Figures A.1 to A.3 showing various head designs) and a rubber needle shield placed on the head design. Accordingly, due to the increased length of the barrel 4 the container 2 may provide a filling volume of 1.9 ml while still having the inner diameter and outer diameter of a standardized syringe with a nominal volume of only 1 ml. In analogy to this one example, however, also other prefilled syringe dimensions as described in the ISO 11040-4 standard can be extended.

The flange portion 10 of the container 2 is manufactured, as far as appropriate, in accordance to the cross-sectional shape and the standardized manufacturing accuracy of a flange of a standardized vial according to the ISO 8362-1 standard. In particular, the flange portion 10 put on top of the cylindrical barrel 4 forms, in a circumferential direction, a continuous circular flange in line with the flange of a conventional vial according to the ISO 8362-1 standard. Insofar, the flange is also in line with a form B of a finger flange of a standardized syringe (cf. ISO 11040-4, Figure 1, Form B). Additionally, the flange portion 10 is formed to comply, in terms of its cross-sectional shape, in particular in terms of its axial length/height, in terms of its upper inner edge, and in terms of its upper end surface, the relevant specifications as indicated in Figure 1, Figure 2, Figure 3 and Table 1 of the ISO 8362-1 standard. Specifically, the axial length/height of the flange portion 10 amounts to 3.6 ±0.2 mm, the bevel angle of the upper inner edge is approximately 45°, and the taper angle of the upper end surface of the flange is 3° ±2° (cf. ISO 8362-1, Figures 1 to 3).

Further, in the example shown in Figure 1 the flange portion 10 has a tapered lower end surface with a taper angle of 10° ±5° similar to a standardized vial (cf. ISO 8362-1, Figures 1 to 3). On the other hand, the flange portion 10 differs, in terms of its inner diameter and outer diameter, from the relevant specifications of the ISO 8362-1 standard and is formed so as to smoothly match the respective barrel dimensions, as it is shown in Figure 1. Instead of the tapered lower end surface seen in Figure 1, the flange portion may have a radially extending flat lower end surface. Unlike a conventional vial, however, the container 2 of a primary packaging according to the present invention has no neck constriction as it is specified in the ISO 8362-1 standard (cf. ISO 8362-1, Figure 1: d3, h3) but it may/can be finished at an inner side as described in the aforementioned standard (cf. ISO 8362-1, Figures 1, 2, 3: neck finish without or with blow back according to Models A, B, C).

As further shown in Figure 1, the primary packaging 1 further comprises a suitable closure element 12 which, in this example, is adapted to tightly fit onto the flange portion 10 and to tightly fit into the barrel 4 through the open second end 6 of the container 2. In this example, the closure element 12 is a stopper that is made of rubber. This closure element 12 has a fitting portion 14 adapted to tightly fit into the barrel 4, and a support portion 16 adapted to rest on the flange portion 8 in a sealing manner. The closure element 12 may meet the cross-sectional shape and relevant dimensions of a stopper that is standardized in the ISO 8362-2 standard, or its dimensions are adapted to more convenient dimensions.

The primary packaging 1 further comprises a flip-off type seal 18 which comprises an aluminum cap 20 which is placed on the closure element 12 fitted to the container 2, which surrounds the closure element 12 and the flange portion 10 of the container 2, and which engages the flange portion 10 of the container 2. The aluminum cap 20 has a central opening 22 which is normally, i.e. during storage, transport, etc., closed by a plastics element 24 and which gives access to the closure element 12 covered by the aluminum cap 20 after removal of the plastics element 24. The plastics element 24 has a flat lower surface 26 resting on the aluminum cap 20. The plastics element 24 is made from an flexible, in this example elastic, material and is releasably fitted to the aluminum cap 20 by positive interaction with a circumferential edge of the central opening 22 of the aluminum cap 20 in such a way that it can be flipped off from the aluminum cap 20 to thereby give access, via the central opening 22, to the closure element 12 placed below the aluminum cap 20 still being engaged to the container 2. In such flip-off state a user may extract a medical or pharmaceutical compound from within the container 2 by means of a (not shown) needle extraction system penetrating a weak membrane portion 17 of the closure element 12, via the central opening 22 of the aluminum cap 20. The seal 18 includes an engaging portion 28 which tightly encloses the closure element 12 in a sealing manner and engages the flange portion 10 of the container 2. As shown in Figure 1, the seal 18 functions as an overseal to secure the closure element 12 to the container 2. In this way, the seal 18 keeps the container 2 and closure element 12 in a sterile condition. By this configuration, the primary packaging 1 functions as a conventional vial.

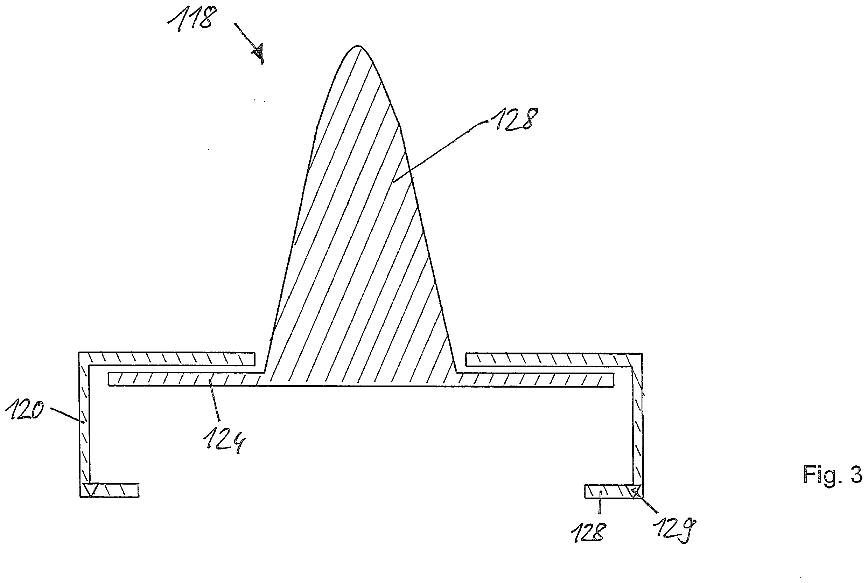

Figure 2 shows, in a sectional side view, another variant of a closure element, which in this example is referenced by reference sign 112 and which is fitted to the container 2 of Figure 1 instead of the closure element 12. In this example, a tear-off type seal 118 comprises an aluminum cap 120 with a central opening 122 and a plastics element 124 having a flat lower surface 126 adapted to rest on a support portion 116 of the closure element 112. Further, the seal 118 comprises a gripping portion 128 having an outwardly extending form. In particular, the gripping portion 128 penetrates the central opening 122 of the aluminum cap 120, as it is seen from Fig. 2, so that the gripping portion 128 can be easily grasped by a user. In the variant, the gripping portion is an integral part of the plastics element 124. Alternatively, the gripping portion 124 may be formed separately of and be securely fitted to the plastics element 124. At an engaging portion 128 of the aluminum cap 120 that engages the flange portion 10 of the container 2 a circumferentially extending, predetermined breaking point 129 (cf. Fig. 3) is disposed such that lifting, folding or snapping the gripping portion 128 causes the major part of the aluminum cap 120 as well as the closure element 112 to be removed from the container 2. To this end, the aluminum cap 120 and the closure element 112 are in tight frictional contact with each other. In this way, the primary packaging 1 functions or may be used as an ampoule.

Figure 3 shows an enlarged, schematized sectional side view of the closure element 112. In particular, the position of the predetermined breaking point 129 at the engaging portion 128 can be clearly seen.

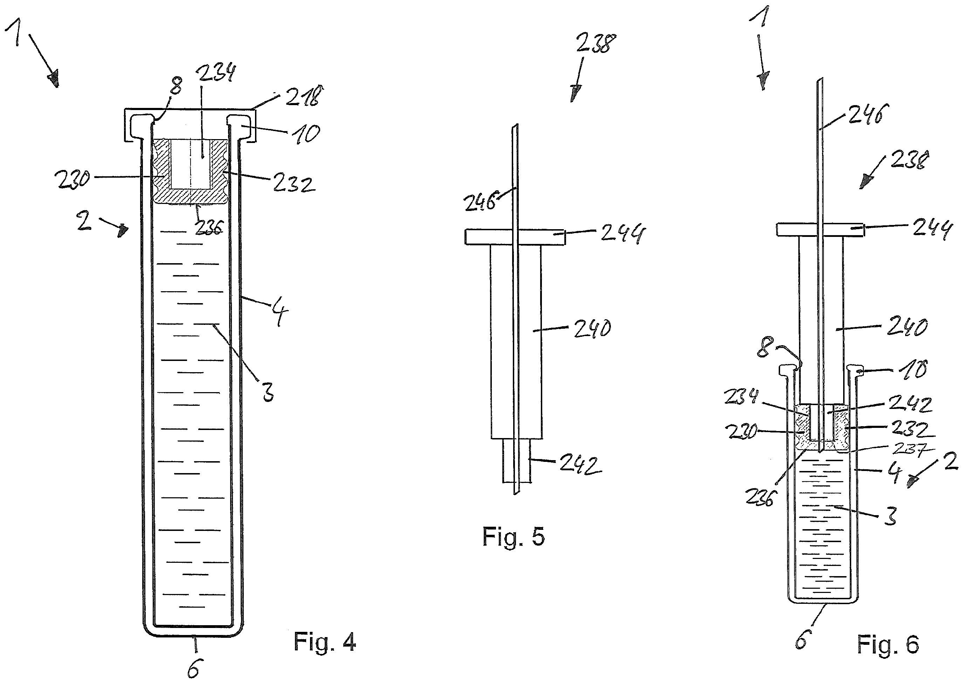

Figure 4 shows an embodiment of a primary packaging according to the present invention in a schematized sectional side view. In this embodiment, a container 2 of the same type as described above with reference to Figs. 1 and 2 is plugged at its open first end 8 by another variant of a closure element which, in the embodiment, is formed as a cylindrical plunger stopper 230. This plunger stopper 230 has a fitting portion 232 adapted to tightly fit into the barrel 4 of the container 2. On its top, which means on the side facing to the open second end 8 of the barrel 4, the plunger stopper 230 has an attachment means 234 which, in this embodiment, is formed as a female thread according to ISO 11040 no. 4.1 b), for screw connection with a needle extraction system 238 shown in Fig. 5. On its bottom, which means on the side facing to the closed first end 6, the plunger stopper 230 has a bottom portion 236 that is adapted to be pierceable by a conventional cannula or a hollow needle as seen from Fig. 5. In the embodiment, the closure element 230 is oversealed by a seal 218 which may correspond in structure to the seal 18 shown in Figure 1. The seal 218 is appropriate in particular if the primary packaging 1 is to be stored, transported, sold, etc. as an intermediate product, i.e. without the needle extraction system 238 being attached to the closure element 230. In case the primary packaging is to be stored, transported, sold, etc. as final product, i.e. with the needle extraction system 238 being attached to the closure element 230, however, it does not include the seal 218, so that the seal 218 may be provided only if necessary or appropriate. In this latter case, if the primary packaging is assembled as a final product the needle extraction system 238 may be half or loosely attached to the closure element 230 so that a weak membrane portion 237 of the closure element 230 is not yet penetrated and is fully or tightly attached to the closure element 230 by the user to penetrate the weak membrane portion 237 of the closure element 230 only at the time of removal of the medical or pharmaceutical compound from within the container 2.

Figure 5 shows a schematized sectional side view of the needle extraction system 238 for removal of the medical or pharmaceutical compounds 3 from within the container 2. The needle extraction system 238 comprises a cylindrical plunger rod element 240 that has an outer diameter which is smaller than the inner diameter of the barrel 4. Also, the plunger rod element 240 has a length that preferably complies with the length of the barrel 4. Further, the plunger rod element 240 further has an attachment means 242 disposed at a first axial end, which is formed as a male thread made of plastic for screw connection with the closure element 230. By such attachment means 242 the plunger rod element 240 is releasably attachable to the attachment means 234 of the plunger stopper 230. On a second axial end, the plunger rod element 240 has a finger flange 244 which extends radially outwards and which is formed so that it can be easily grasped by a user.

The plunger rod element 240 further comprises a cannula 246 which extends through the whole plunger rod element 240. That means that the plunger rod element 240 is protruded by the cannula 246 on both axial ends. In this embodiment, the cannula 244 is formed as a hollow needle that is securely fixed to the plunger element 240. Alternatively, however, the cannula 246 can be integrally formed to the plunger rod element 240. Further, the cannula 246 is adapted to pierce the bottom portion 236 of the plunger stopper 230. When the needle extraction system 238 is attached to the plunger stopper 230 and is moved in an axial direction towards the first end 8 of the barrel 4, the medical or pharmaceutical compounds 3 can be removed from the container 2. To restrict the amount of compound 3 remaining in the container after having fully operated the needle extraction system 238, the protrusion of the cannula 246 from a lower side of the plunger rod element 240 is minimized as short as possible. In this way, the primary packaging functions as a (pre-filled) syringe.

Figure 6 shows a schematized sectional side view of the primary packaging when the needle extraction system 238 is fitted to the plunger stopper 230. For attaching the needle extraction system 238 to the plunger stopper 230 seal 218, if available, has to be firstly removed.

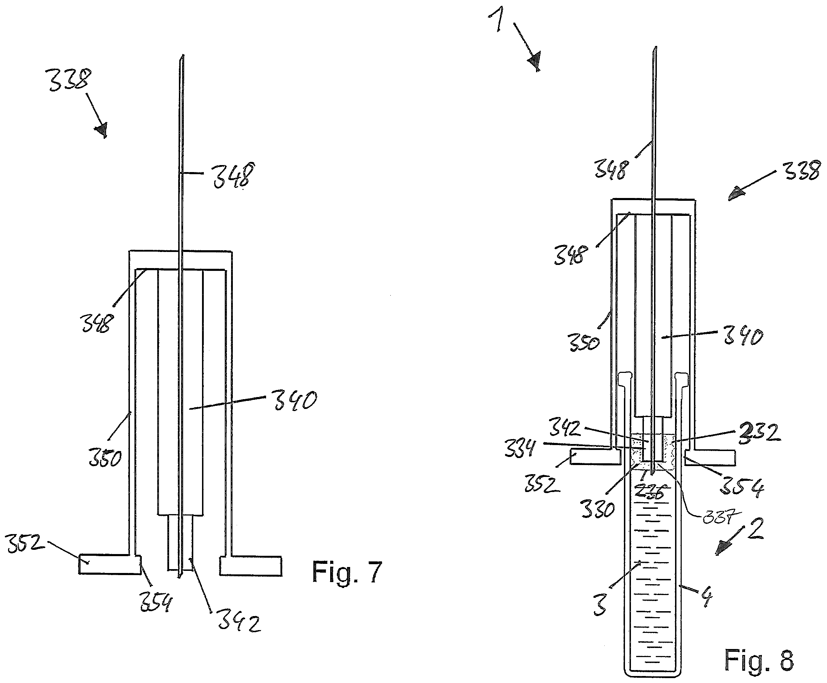

In Figure 7, a modification of the needle extraction system is shown in a schematized sectional side view. The needle extraction system according to the modification is referenced by the reference sign 338 and comprises a radially extended abutting portion 348 which is integrally formed to a first axial end of a plunger rod element 340. Alternatively, the abutting portion 348 can be e. g. releasably attached to the first axial end of the plunger rod element 340. The plunger rod element has an outer diameter which is smaller than the inner diameter of the barrel 4 and comprises an attachment means 342 which complies with the attachment means 242 as described with reference to Figs. 5 and 6. The needle extraction system 338 further comprises a cannula 346 which extends through the whole plunger rod element 340.

The needle extraction system 338 of Figure 7 comprises a cylindrical tube section 350 which extends substantially coaxially with the plunger rod element 340 from the abutting portion 348 in a direction of a second axial end of the plunger rod element 340 facing away from its first axial end. Adjacent to the second axial end of the plunger rod element 340 the tube section 350 has a radially outwardly extending finger flange 352 that can be easily grasped by a user. The finger flange 352 has a radially inwardly extending projection 354 which is configured to slide along the outer surface of the container 2. To assemble the needle extraction system 338 to the container 2, the tube section 350 may be, within limits, elastically deformable such to that its end portion including the finger flange 352 is slightly radially expandable.

By this configuration, as shown in Figure 7, the plunger rod element 340 can be placed over the barrel 4 of the container 2. The lengths of the plunger rod element 340 and of the tube section 350 are adapted to the inner length of the barrel 4 so that a minimum stroke of the plunger rod element 340 or a minimum way of the tube section 350, respectively, corresponds to the inner length or depth of the barrel 4.

Figure 8 shows a schematized sectional side view of a primary packaging 1 having the needle extraction system 338 fitted to a plunger stopper 330, wherein the plunger stopper corresponds to the plunger stopper 230 as described above. It can be clearly seen that the lengths of the plunger rod element 340 and of the tube section 350 comply with the length of the barrel 4. With the projection 354 of the tube section 350, the needle extraction system 338 can be used as a disposable syringe.To summarize, by the configuration according to the present invention, a modular system for assembling a primary packaging is provided, which is based on a single type of container 2 that preferably meets, in terms of the cross-sectional shape and dimensions of the barrel 4, the relevant specifications of the barrel of a standardized syringe and preferably meets, in terms of the cross-sectional shape at least with respect to its upper end, the relevant specifications of the flange of a standardized vial. In particular, the barrel 4 is preferably formed to comply with the ISO 11040-4 standard specifications in terms of the barrel diameters d1, d2, and d5 and the barrel wall thickness s1 as indicated in Figure 1 and Tables 1 and B.1, respectively, of the ISO 11040-4 standard. Further, the flange portion 10 is preferably formed to comply with the ISO 8362-1 standard specifications at least in terms of the upper end cross-sectional shape. On the other hand, in terms of the inner diameter and outer diameter, the flange portion 10 may differ from the respective specifications of the ISO 8362-1 standard and may be designed so as to match the barrel dimensions. Thus, the container 2 is formed by the barrel 4 and a flange portion 10 matching the barrel. Accordingly, the container 2 does not have any such neck constriction as a conventional vial.

Further, the closure element is selected from a group of variants of closure elements according to the user's needs and desired type of administration of the medical or pharmaceutical compound stored in the container. The group of variants of closure elements comprises a stopper comprising a fitting portion adapted to tightly fit into the barrel and a support portion adapted to rest on the flange portion (cf. Fig. 1), and a cylindrical plunger stopper comprising a fitting portion adapted to tightly fit into the barrel of the container and adapted to cooperate with an optionally provided needle extraction system (cf. Fig. 2). Other types of closure elements may be added to the group of variants.

Based on the illustrated embodiments, the primary packaging according to the present invention may be modified in many ways. For example, the above described screw fit attachment means 232, 242 of Fig. 6 or 332, 342 of Fig. 8 may be replaced by a conventional snap fit connection according to ISO 11040-5 no. 4.1 a).

Further, in any of the above described embodiments an inner surface of the container 2, which is in contact with the medical or pharmaceutical compound stored in the container 2, may be refined by way of subjecting the inner surface to an appropriate surface finishing process and/or coating process. For example, the inner surface may be treated by diverse plasma treatments or siliconized by baked-on or spray siliconization using silicone oil suspensions or silicone oil. Such treatment is known with conventional prefilled syringes in order to enhance the lubrication property of the inner surface of a syringe barrel or in vials to reduce the extractable volume losses. It has to be noted, however, that according to the present invention a surface finishing of the inner surface of the container is not restricted to the use of the primary packaging as a syringe, but is open to any type of use of the primary packaging whether it is as a syringe, vial or ampoule, which types of use will be described in the following.

## Claims

1. Primary packaging (1) for storage and/or administration of medical or pharmaceutical compounds, said primary packaging (1) comprising:
- a container (2), having a predetermined filling volume for receiving a medical or pharmaceutical compound (3), the container (2) being permanently closed at a first end (6) and having an integrally formed, circumferential flange portion (10) that extends radially at an open second end (8), the container having a cylindrical barrel (4) that extends between the first end (8) and the flange portion (10),
- a closure element (230, 330) for sealing the open second end (8) of the container (2), said closure element (230, 330) being a cylindrical plunger stopper (230, 330) comprising a fitting portion (232, 332) adapted to tightly fit into the barrel (4) of the container (2), and
- a needle extraction system (238, 338) for removal of the medical or pharmaceutical compound from within the container (2), the needle extraction system (238, 338) being adapted to cooperate with the plunger stopper (230, 330) and comprises a plunger rod element (240, 340) which has a finger flange (244, 352) and a cannula (246, 346), wherein
- the flange portion (10) of the container (2) is formed to function as a finger flange, **characterised in that**
- the plunger rod element (240, 340) of the needle extraction system (238, 338) is releasably attachable to the plunger stopper (230, 330) by a snap fit connection or a screw connection (234, 334) and comprises a cannula (246, 346) which extends through the whole plunger rod element (240, 240).

2. Primary packaging according to claim 1, wherein the container (2) meets, in terms of shape and dimensions of the barrel (4), selected specifications of ISO 11040-4 standard for the barrel of a prefilled syringe.

3. Primary packaging according to any one of the preceding claims, wherein the container (2) meets, in terms of the cross-sectional shape of the flange portion (10), selected specifications of ISO 8362-1 standard for the flange of an injection vial.

4. Primary packaging according to any of the preceding claims, wherein the closure element (12, 1 12, 230) meets, in terms of the cross-sectional shape, selected specifications of ISO 8362-2 standard for the closure of an injection vial.

## Patentansprüche

1. Primärverpackung (1) zum Speichern und/oder Verabreichen von medizinischen oder pharmazeutischen Verbindungen, wobei die Primärverpackung (1) umfasst:
- einen Behälter (2) mit einem vorbestimmten Füllvolumen zum Aufnehmen einer medizinischen oder pharmazeutischen Verbindung (3), wobei der Behälter (2) an einem ersten Ende (6) dauerhaft verschlossen ist und an einem zweiten Ende (8) einen einteilig mit ihm ausgebildeten und sich radial erstreckenden Umfangsflanschabschnitt (10) umfasst, wobei der Behälter einen zylindrischen Hohlraum (4) umfasst, der sich zwischen dem ersten Ende (8) und dem Flanschabschnitt (10) erstreckt,
- ein Verschlusselement (230, 330) zum dichten Verschließen des offenen zweiten Endes (8) des Behälters (2), wobei das Verschlusselement (230, 330) ein zylindrischer Kolbenstopfen (230, 330) ist, der einen Passabschnitt (232, 332) umfasst, der fest in den zylindrischen Hohlraum (4) des Behälters (2) einpassbar ist, und
- ein Nadelextraktionssystem (238, 338) zum Entnehmen der medizinischen oder pharmazeutischen Verbindung aus dem Behälter (2), wobei das Nadelextraktionssystem (238, 338) dazu geeignet ist, mit dem Kolbenstopfen (230, 330) zusammenzuwirken, und ein Kolbenstangenelement (240, 340) mit einem Fingerflansch (244, 352) und einer Kanüle (246, 346) umfasst,
**dadurch gekennzeichnet, dass**:
- der Flanschabschnitt (10) des Behälters (2) als Fingerflansch dient, und
- das Kolbenstangenelement (240, 340) des Nadelextraktionssystems (238, 338) durch eine Schnappverbindung oder eine Schraubverbindung (234, 334) lösbar an dem Kolbenstopfen (230, 330) befestigbar ist.

2. Primärverpackung nach Anspruch 1, wobei der Behälter (2) bezüglich Form und Abmessungen des zylindrischen Hohlraums (4) ausgewählten Spezifikationen gemäß dem Standard ISO 11040-4 für den zylindrischen Hohlraum einer vorgefüllten Injektionsspritze genügt.

3. Primärverpackung nach einem der vorhergehenden Ansprüche, wobei der Behälter (2) bezüglich der Querschnittsform des Flanschabschnitts (10) ausgewählten Spezifikationen gemäß dem Standard ISO 8362-1 für den Flansch einer Injektionsampulle genügt.

4. Primärverpackung nach einem der vorhergehenden Ansprüche, wobei das Verschlusselement (12, 1 12, 230) bezüglich der Querschnittsform ausgewählten Spezifikationen gemäß dem Standard ISO 8362-2 für den Verschluss einer Injektionsampulle genügt.

## Revendications

1. Emballage primaire (1) pour le stockage et/ou l'administration de composés médicinaux ou pharmaceutiques, ledit emballage primaire (1) comprenant :
- un récipient (2), ayant un volume de remplissage prédéterminé pour recevoir un composé médicinal ou pharmaceutique (3), le récipient (2) étant fermé en permanence à une première extrémité (6) et ayant une partie de rebord circonférentiel (10) formée en une seule pièce qui se prolonge radialement au niveau d'une seconde extrémité ouverte (8), le récipient ayant un corps cylindrique (4) qui se prolonge entre la première extrémité (8) et la partie de rebord (10),
- un élément de fermeture (230, 330) pour fermer hermétiquement la seconde extrémité ouverte (8) du récipient (2), ledit élément de fermeture (230, 330) étant un bouchon de piston cylindrique (230, 330) comprenant une partie d'ajustement (232, 332) adaptée pour s'ajuster serrée dans le corps (4) du récipient (2), et
- un système d'extraction par aiguille (238, 338) pour retirer le composé médicinal ou pharmaceutique à partir de l'intérieur du récipient (2), le système d'extraction par aiguille (238, 338) étant adapté pour coopérer avec le bouchon de piston (230, 330) et comprend un élément de tige de piston (240, 340) qui comporte un rebord pour doigt (244, 352) et une canule (246, 346),
dans lequel
- la partie de rebord (10) du récipient (2) est formée pour jouer le rôle de rebord pour doigt, **caractérisé en ce que**
- l'élément de tige de piston (240, 340) du système d'extraction par aiguille (238, 338) peut être attaché amovible au bouchon de piston (230, 330) par connexion par encliquetage ou par connexion par vis (234, 334) et comprend une canule (246, 346) qui se prolonge à travers la totalité de l'élément de tige de piston (240, 240).

2. Emballage primaire selon la revendication 1, dans lequel le récipient (2) satisfait, en ce qui concerne la forme et les dimensions du corps (4), des spécifications sélectionnées de la norme ISO 11040-4 pour le corps d'une seringue préremplie.

3. Emballage primaire selon l'une quelconque des revendications précédentes, dans lequel le récipient (2) satisfait, en ce qui concerne la forme en coupe transversale de la partie de rebord (10), des spécificités sélectionnées de la norme ISO 8362-1 pour le rebord d'un flacon pour injection.

4. Emballage primaire selon l'une quelconque des revendications précédentes, dans lequel l'élément de fermeture (12, 1 12, 230) satisfait, en ce qui concerne la forme en coupe transversale, des spécifications sélectionnées de la norme ISO 8362-2 pour la fermeture d'un flacon pour injection.
